# EUROPEAN PATENT APPLICATION

(11) **EP 2 641 592 A1**
(43) Date of publication of application: **25.09.2013**
(21) Application number: 12160946.5
(22) Date of filing: 23.03.2012
(51) Int. Cl.: A61K 9/14, A61K 31/4184, A61P 35/00

(54) **Pharmaceutical formulation comprising bendamustine**

(71) Applicant: Salmon Pharma GmbH, 4002 Basel (CH)
(72) Inventor: The designation of the inventor has not yet been filed
(74) Representative: Isarpatent

(57) **Abstract**

The present invention is related to the field of pharmaceutical compositions for the treatment of various disease states, particularly neoplastic diseases and autoimmune diseases. It especially relates to the oral application of bendamustine and its derivatives. The invention further relates to a process for preparing a pharmaceutical composition which comprises bendamustine and its derivatives prepared by hot melt extrusion using pharmaceutically acceptable excipients. The invention also relates to such pharmaceutical compositions and hot melt extrudates.

## Description

### Field of the invention

The present invention is related to the field of pharmaceutical compositions for the treatment of various disease states, particularly neoplastic diseases and autoimmune diseases. It especially relates to the oral application of bendamustine and its derivatives. The invention further relates to a process for preparing a pharmaceutical composition which comprises bendamustine and its derivatives prepared by hot melt extrusion using pharmaceutically acceptable excipients. The invention also relates to such pharmaceutical compositions and hot melt extrudates.

### Background of the invention

Nitrogen mustards are highly reactive molecules and especially unstable in water. Thus the pharmaceutical forms of such molecules are hard to formulate and usually supplied as lyophilized form for injection which then need to be reconstituted by the hospital personal prior to injection. This step is realised with water for injection and once the molecule is dissolved in water it needs to be directly used because of the stability problems. Thus a stable oral formulation will for sure increase patient compliance as well as will avoid the problems faced during the application.

Bendamustine 4-[5-[Bis(2-chloroethyl)amino]-1-methylbenzimidazol-2-yl]butanoic acid is a nitrogen mustard containing a benzimidazole ring whose structure includes an active nitrogen mustard.

Presently, bendamustine is supplied as lyophilized powder in a brown glass due to its light instability. This lyophilized powder needs to be reconstituted prior to injection as it is the case for all nitrogen mustards. There are two different dose strengths of bendamustine, 100 mg and 25 mg. Vials are opened and 100 mg dose is reconstituted with 40 mL water for injection and 25 mg dose is reconstituted with 10 mL water for injection as close as possible to the time of administration. Furthermore these reconstituted solutions are diluted in 500 mL 0.9% sodium chloride for injection. The route of administration is by intravenous injection over 30 or 60 minutes. Once the bendamustine solution is prepared it should be given to the patient within 7 hours of vial reconstitution. Due to these stability issues, to increase the patient compliance and to overcome the difficulty due to the fact that patients are needed to be hospitalized for the administration, an oral formulation of bendamustine would be desirable.

Although bendamustine is highly orally bioavailable, there have been up to date no oral formulations of this compound. This is most probably due to high variations in oral bioavailability. In previous studies of Preis et. al. (Pharmazie 1985, 40), intravenous and oral applications of bendamustine were realized in a dose range of 4.2-5.5 mg/kg in humans. 25 mg of commercially available product Cytostasan (for intravenous applications) was filled into capsules and used as the oral formulation. As a result of pharmacokinetic studies tₘₐₓ was found to be 1 hour and mean oral bioavailability was calculated as 57% with a coefficient of variation of 44%.

In another study by Weber (Pharmazie 1991, 46:8), oral bioavailability of bendamustine was investigated in mice and found to be 40 %.

The high variations in oral bioavailability of bendamustine are most probably due to the low solubility of bendamustine in pH < 5.0 and low stability pH > 5.0. Thus, absorption of bendamustine takes place where it is not stable resulting in high variations in bioavailability. A new oral formulation which would realise the solubility of bendamustine at low pHs and allow the absorption of this compound from early gastrointestinal tract where pH is lower than 5.0 will decrease the variations and increase the oral bioavailability allowing the oral application of this compound.

There are different patent applications available related to the oral application of bendamustine and in all of these patents solubility and stability of bendamustine is increased through excipients with conventional formulation techniques:
In patent application WO2010/063493 the formulation strategy is described as comprising bendamustine or a pharmaceutically acceptable ester, salt or solvate thereof as an active ingredient and at least one pharmaceutically acceptable excipient which compositions are said to have a good stability and an improved dissolution profile. The pharmaceutical excipient is more specifically a non-ionic surfactant which increases the solubility of bendamustine after oral application.
In patent application WO2010/126676, the oral formulation of bendamustine is realised through combination of bendamustine with one or more non-aqueous pharmaceutically acceptable excipients selected from the group of solvents and cosolvents. The formulation preferably has the form of a solid solution, solid suspension, solid dispersion, liquid dispersion, suspension, emulsion, microemulsion, gel or solution. However, it is not shown therein that the bendamustine formulation has a sufficiently uniform and reliable bioavailability.
In patent application US20120003309, an oral formulation of bendamustine was realised with addition of pharmaceutically acceptable saccharide derivatives. Although stability of these formulations was shown, there is not sufficient information of uniform or higher bioavailability given in this application.
In patent application W02011/151086, higher dissolution rates of the oral formulations of bendamustine were realised through incorporating surfactant excipients in the formulations. Although the coefficient of variation was decreased after the oral application there was found to be no increase in the bioavailability of the substance.
In patent application WO 2011/151087 liquid surfactants were used in order to increase the stability and bioavailability.

Pharmaceutical compositions comprising an active ingredient molecularly dispersed or dissolved in one or more polymeric carriers have been described as molecular dispersions, solid solutions or glass solutions. In general, in these types of formulations using hot melt extrusion technique, crystalline substance is *in-situ* dissolved in one or more polymers forming amorphous structures.

In view of the existing technologies, it is the object of the invention to provide a pharmaceutical composition comprising bendamustine suitable for oral administration. Particularly, it is the object of the present invention to provide a pharmaceutical composition comprising bendamustine for oral administration which provides a high bioavailability, low variation in oral bioavailability and increased stability. Further, it is the object of the invention to provide a pharmaceutical composition comprising bendamustine showing an increased solubility of bendamustine at a pH < 5.0 as well as an increased stability at a pH of > 5.0.

### Summary of invention

In order to achieve the above stated objects, a hot-melt extruded pharmaceutical dosage form with immediate release of bendamustine embedded in a matrix comprising at least one water soluble or water insoluble polymer or combination thereof is provided by the present invention. Surprisingly, it turned out that these hot melt extrusion formulations are suitable for oral administration and provide a good bioavailability and increased stability compared to prior art formulations. Furthermore the present invention discloses a method for producing hot melt extrusion formulations of bendamustine.

The inventors recognized that hot melt extrusion formulations of bendamustine increase the dissolution rate of bendamustine in the stomach and enable the absorption of this drug in early segments of the intestines offering an increased bioavailability and decreased inter-individual variability. Furthermore, the stability of bendamustine is increased through hot melt extrusion formulations making it appropriate for the oral applications.

### Detailed description of invention

As described above and further in the present application, the inventors have surprisingly found out that hot melt extrudates of bendamustine do not only have a better solubility at a pH < 5.0, but also a better bioavailability together with an increased stability at pH values > 5.0.

According to a first aspect, the present invention is directed to pharmaceutical formulations comprising bendamustine or a derivative thereof as an active pharmaceutical ingredient (API) characterized in that the API is embedded in a pharmaceutically acceptable polymer matrix. The pharmaceutical composition preferably is produced by a hot-melt extrusion process resulting in extruded products which in turn will be processed to the final pharmaceutical product such as a tablet. For example, granulates may be formed by hot-melt extrusion which may be milled subsequently and compressed (along with further excipients/auxiliaries) into tablets or may be filled into capsules.

Hot-melt extrusion (HME) is the process of transferring a powder blend of drug and carrier by a rotating screw through the heated barrel of an extruder and pressing the melt through a die into a product of uniform shape. Hot-melt extrusion thus can be simply defined as the process of forming a new material (the extrudate) by forcing it through an orifice or die under controlled conditions, such as temperature, mixing, feed-rate and pressure. Specifically, HME dosage forms are complex mixtures of API, polymers, excipients, and further auxiliaries which are blended using industry-standard equipment. The mixture is processed at elevated temperature and pressure, which disperses the drug in the matrix at a molecular level through the formation of a solid solution. The extruded material can be further processed into a variety of dosage forms, including capsules, tablets and transmucosal systems.

HME offers many advantages compared with traditional processing techniques that have been typically used to produce oral solid dosage forms. In particular, solvents are not required, which makes the process more environmentally friendly and cost effective. The end product (or extrudate) often is referred to as "solid dispersion" or "solid solution".

The term "derivative" as used in the present application in connection with bendamustine in particular is to be understood as the pharmaceutically acceptable salt, ester or solvate form of bendamustine.

As used herein, "pharmaceutically acceptable salts" refer to derivatives of the disclosed compounds wherein the parent compound is modified by making salts thereof. For example, such conventional salts include, but are not limited to, those derived from inorganic acids such as hydrochloric, hydrobromic, sulfuric, sulfamic, phosphoric, nitric acid to name a few. Bendamustine HCl is in particular preferred.

The pharmaceutical composition of the present invention incorporates a polymer which is a water soluble polymer, a water insoluble polymer or a combination thereof.

The water soluble polymers according to the present invention preferably include homopolymers and co-polymers of N-vinyl lactams, especially homopolymers and copolymers of N-vinyl pyrrolidone, e.g., polyvinylpyrrolidone (PVP), co-polymers of PVP and vinyl acetate, co-polymers of N-vinyl pyrrolidone and vinyl acetate or vinyl propionate, dextrins such as grades of maltodextrin, cellulose esters and cellulose ethers, high molecular polyalkylene oxides, such as polyethylene oxide and polypropylene oxide, and co-polymers of ethylene oxide and propylene oxide. The bendamustine to water soluble polymer ratio can be in between 1:0.5 to 1:100 by weight, preferably 1:1 to 1:9.

The water insoluble polymers that can be used according to the present invention include acrylic copolymers, e.g., Eudragit^{®} E100 or Eudragit^{®} EPO; Eudragit^{®} L30D-55, Eudragit^{®} FS30D, Eudragit^{®} RL30D, Eudragit^{®} RS30D, Eudragit^{®} NE30D, Acryl-Eze^{®} (Colorcon Co.); polyvinylacetate, for example, Kollicoat^{®} SR 30D (BASF Co.); cellulose derivatives such as ethylcellulose, cellulose acetate, e.g., Surelease (Colorcon Co.), Aquacoat ECD and Aquacoat CPD (FMC Co.). The most preferred water insoluble polymer is Eudragit^{®} E100. Water insoluble polymer to Bendamustine ratio can be in between 0.5:1 to 100:1 by weight preferably 1:1 to 9:1.

The pharmaceutical composition of the present invention additionally may contain one or more pharmaceutically acceptable excipients and/or auxiliaries.

The excipients that can be used in the further formulation of extrudates are chemically stable and nonreactive with bendamustine, or its salts, esters/solvates, under one or more of the storage conditions described herein. Excipients suitable for use in the present invention include those identified by the U.S. Food and Drug Administration as Generally Regarded as Safe (GRAS). Preferred excipients for use in the invention include solvents and co-solvents such as, for example, propylene carbonate, propylene glycol, and polyethylene glycols (for example, PEG 1000 and PEG 1500, PEG 1450, surfactants and co-surfactants such as, for example, medium chain monoglycerides such as, for example, glyceryl caprylate, glyceryl monolaurates, polyethylene glycol hydroxy stearates, polysorbates (for example, polysorbate 80), polyethylene-polypropylene glycol copolymers (for example, Poloxamer 188), tocopherol polyethylene glycol 1000 succinate, triglycerides (for example, corn oil, including super refined corn oil), and polyethylene glycol stearates, polyethylene glycol laurates, for example polyethylene glycol mono-and dilaurate mixtures, to name a few. Disintegrants, diluents, lubricants, glidants, emulsifying-solubilizing agents, sweetening agents, coating agents, antimicrobial preservatives, and the like, are also within the scope of the invention.

The amount of each excipient used within the scope of the invention will vary, depending on the particular excipients selected. Preferably, the pharmaceutical compositions of the invention will include at least one pharmaceutically acceptable excipient.

Applicable solid excipients can include one or more substances that may also act as flavoring agents, lubricants, solubilizers, suspending agents, fillers, glidants, compression aids, binders or tablet-disintegrating agents or an encapsulating material. In powders, the excipient is a finely divided solid that is in admixture with the finely divided active ingredient, i.e. bendamustine or a pharmaceutically acceptable salt thereof. In tablets, the active ingredient is mixed with an excipient having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Suitable drug dosage forms include, but are not limited to, tablets, for example, immediate-, controlled-, and extended-release tablets, pills, capsules, soft gels, sachets, granules, powders, chewing gums, solid suspensions, emulsions, and solutions.

Liquid excipients may be used in preparing solutions, suspensions, emulsions, syrups, and elixirs. Extrudates comprising bendamustine or a pharmaceutically acceptable salt, ester or solvate thereof, can be suspended in a pharmaceutically acceptable liquid excipient such as an organic solvent or pharmaceutically acceptable oils or fat. The liquid excipient can contain other suitable pharmaceutical additives such as solubilizers, emulsifiers, buffers, preservatives, sweeteners, flavoring agents, suspending agents, thickening agents, colors, viscosity regulators, stabilizers, or osmo-regulators. Suitable examples of liquid excipients for oral administration alcohols (including monohydric alcohols and polyhydric alcohols e.g. glycols) and their derivatives, oils (e.g. fractionated coconut oil and arachis oil), and for short contact periods, water (particularly containing additives as above, e.g. cellulose derivatives, preferably sodium carboxymethyl cellulose solution).

Examples of auxiliaries are plasticizers which can be used in order to lower the glass transition of the polymers include, but are not limited to, polysorbates such as sorbitan monolaurate (Span 20), sorbitan monopalmitate, sorbitan monostearate, sorbitan monoisostearate; citrate ester type plasticizers like triethyl citrate, citrate phthalate; propylene glycol; glycerin; polyethylene glycol (low and high molecular weight); triacetin; dibutyl sebacate, tributyl sebacate; dibutyltartrate, dibutyl phthalate. The plasticizers are in generally included in the formulation in 10 % of the polymer amount.

Furthermore antioxidants and other stabilizing agents can be included in the formulations. These substances include but not limited to ascorbic acid, butylhydroxyanisole (BHA), butylhydroxytoluene (BHT), salts of ascorbic acid, monothioglycerol, phosphorous acid, vitamin C, vitamin E and the derivatives thereof, coniferyl benzoate, nordihydroguajaretic acid, gallus acid esters, sodium bisulfite, particularly preferably butylhydroxytoluene or butylhydroxyanisole and α-tocopherol.

In an embodiment, the pharmaceutical composition of the invention contains one or more additional active pharmaceutical ingredients (API's) and/or pharmaceutically acceptable auxiliaries. Those API's may be preferably selected from etoposide, fludarabine, mitoxantrone, methotrexate, prednisone, rituximab, vincristine and/or 90Y-ibritumomab tiuxetan.

The extrudates formed by hot-melt extrusion can be designed to be used in variety of different pharmaceutical compositions and formulations such as oral delivery as tablets, capsules or suspensions, pulmonary and nasal delivery, topical delivery as emulsions, ointments and creams, and parenteral delivery as suspensions, microemulsions or as a depot. Oral delivery is most preferred.

Preferably the pharmaceutical composition is in single unit dosage form, e.g. as tablets, capsules, powders, solutions, suspensions, emulsions, or granules. In such form, the composition is sub-divided in unit dose containing appropriate quantities of the active ingredient; the unit dosage forms can be packaged compositions, for example packaged powders, vials, ampoules, and the like. The unit dosage form can be, for example, a capsule or tablet itself, or it can be the appropriate number of any such compositions in package form.

The daily dosage of bendamustine might be, but is not limited to the range of 50 to about 1000 mg/day and most preferably 100 to about 500 mg of the active ingredient. A single dose may vary between 25 mg and 100 mg of bendamustine.

A further aspect of the present invention is the use of the pharmaceutical composition of the present invention in the treatment of neoplastic or autoimmune diseases. Neoplastic diseases, which may be treated comprise chronic lymphocytic leukemia, Hodgkin's disease, non-Hodgkin's lymphoma, multiple myeloma and lung cancer.

According to a further aspect, the pharmaceutical composition of the present invention may be prepared through hot melt extrusion technique which involves hot melt extrusion of bendamustine with one or more water soluble or water insoluble polymer or a combination thereof using a conventional extruder as known to a person skilled in the art.

As such, the method of producing a pharmaceutical composition comprising bendamustine or a derivative thereof as an API comprises the steps of:
- mixing and melting the API and a polymer in order to provide a melt,
- extruding the melt through a die thereby forming an extrudate; and
- cooling the extrudate until it solidifies.

In general, hot melt extrusion technique includes steps of preparing a homogenous melt of bendamustine or its pharmaceutically approved derivatives, the polymer and the excipients followed by cooling the melt until it solidifies. Typically one component will melt dissolving the other components and forming a solid solution. Mixing of the components can take place before, after or during the melt.

Usually the melt temperature is in the range of about 60 °C to 240 °C, preferably about 80 °C to 140 °C. Different extruders can be used for this process for example but not limited to single screw extruders or multiscrew extruders. Extrudates can have different shapes such as but not limited to rhodes, granulates, tubes or beads and can be further processed into any desired shape as outlined above.

In a further aspect, the present invention discloses a pharmaceutical composition obtainable by the above method and its oral, topical, pulmonary, nasal, or parenteral use. Furthermore, the pharmaceutical composition of the invention may be used in the treatment of neoplastic or autoimmune diseases.

The present application now is described in more detail by the following Examples. However, it is noted that the Examples are provided for illustrative purposes only and should not be construed to limit the scope of the invention in any way.

### Examples

### Example 1

Preparation of hot melt extrudates

| | **F1** | **F2** | **F3** | **F4** | **F5** | **F6** | **F7** | **F8** |
|---|---|---|---|---|---|---|---|---|
| **Bendamustine** | 20 % | 20 % | 20 % | 20 % | 20 % | 20 % | 20 % | 20 % |
| **Eudragit^{®} E 100** | 70 % | - | 80 % | - | 63 % | - | 73 % | - |
| **Plasdone S630** | - | 70 % | - | 80 % | - | 63 % | - | 73 % |
| **PEG 1000** | 10% | 10% | - | - | 10% | 10% | - | - |
| **Butylhydroxytoluene** | - | - | - | - | 2 % | 2 % | 2 % | 2 % |
| **Ascorbic acid** | - | - | - | - | 5% | 5% | 5% | 5% |

Bendamustine and excipients are physically mixed and extruded using a twin screw extruder in temperatures between 90 °C - 150 °C. After the hot melt extrusion granulates are milled to fine powder for further formulation. Powder extrudes were either filled in hard gelatine capsules or compressed into tablets with suitable excipients

### Example 2

Tablet formulations were prepared as follows

| Receipt: | |
|---|---|
| Bendamustine extrudate | 75 % |
| Avicel PH 102 | 19 % |
| Plasdone X1-10 | 4 % |
| Mg stearate | 1 % |
| Aerosil 200 | 1 % |

Bendamustine extrudate is mixed with Avicel PH 102 and Plasdone X1-10. This mixture is further mixed with Aerosil 200 and Mg Stearate and compressed into tablets using for instance, a 0.738"x0.344" oval shaped punch.

### Example 3

### Coating of tablets

Tablets can be coated using a dispersion of Opadry white. Kernels were placed in a coating pan heated up to 40 °C and coated until a weight gain of 20 %.

### Example 4

Dissolution studies were conducted with extrudates filled in hard gelatine capsules and 100 % dissolution was observed in less than 20 minutes as measured with paddle apparatus at 50 rpm according to the European Pharmacopoeia in 500 mL of a dissolution medium at a pH of 1.2.

| | **5 min** | **10 min** | **15 min** | **20 min** |
|---|---|---|---|---|
| **F1** | 72.99 | 89.2 | 95.8 | 99.22 |
| **F2** | 83.55 | 91.7 | 100.5 | 100.5 |

### Example 5

Assay and impurity analyses of extrudates were conducted with HLPC-UV methods validated in accordance with cGMP regulations.

| Stability of extrudates at 25 °C 60% RH | | | | |
|---|---|---|---|---|
| | **Assay 0. month [%]** | **Assay 1. month [%]** | **Total impurity 0. month [%]** | **Total impurity 1. month [%]** |
| **F1** | 99.6 | 99.4 | 0.32 | 0.34 |
| **F2** | 98.5 | 98.7 | 0.27 | 0.32 |
| **F3** | 100.1 | 99.6 | 0.39 | 0.35 |
| **F4** | 99.4 | 100.0 | 0.32 | 0.34 |
| **F5** | 99.4 | 98.7 | 0.28 | 0.32 |
| **F6** | 99.8 | 100.2 | 0.30 | 0.32 |
| **F7** | 99.3 | 99.7 | 0.34 | 0.29 |
| **F8** | 100.2 | 99.4 | 0.25 | 0.29 |

| Stability of extrudates at 40 °C 75% RH | | | | |
|---|---|---|---|---|
| | **Assay 0. month** | **Assay 1. month** | **Total impurity 0. month** | **Total impurity 1. month** |
| **F1** | 99.6 | 99.5 | 0.32 | 0.42 |
| **F2** | 98.5 | 97.9 | 0.27 | 0.38 |
| **F3** | 100.1 | 99.6 | 0.39 | 0.44 |
| **F4** | 99.4 | 99.2 | 0.32 | 0.39 |
| **F5** | 99.4 | 99.6 | 0.28 | 0.35 |
| **F6** | 99.8 | 100.1 | 0.30 | 0.38 |
| **F7** | 99.3 | 98.7 | 0.34 | 0.36 |
| **F8** | 100.2 | 99.5 | 0.25 | 0.34 |

### Example 4

### Bioavailability of extrudates

*In-vivo* bioavailability tests were done with rats. Formulation 1 and formulation 2 was given as suspension in PEG solution. Furthermore bendamustine powder was also given orally and parenteral for the calculation of absolute bioavailability. Samples were taken at 0, 0.5, 1, 2, 4, 8 hours and quantified with HPLC/MS/MS after extraction of Bendamustine from plasma.

Formulations containing Bendamustine extrudates showed a bioavailability of 42 % whereas powder form was only 33 % bioavailable.

## Claims

1. A pharmaceutical composition comprising bendamustine or a derivative thereof as an active pharmaceutical ingredient (API)
**characterized in that**
the API is embedded in a pharmaceutically acceptable polymer matrix.

2. The pharmaceutical composition of claim 1, wherein the API is finely dispersed or dissolved in the polymer matrix.

3. The pharmaceutical composition of claim 1 or 2, wherein the polymer is a water soluble polymer, water insoluble polymer or a combination thereof.

4. The pharmaceutical composition according to any of claims 1 to 3, wherein the weight ratio of the API to the polymer is 1:0.5 to 1:100.

5. The pharmaceutical composition of any of claims 1 to 4, wherein the composition contains one or more additional API's and/or pharmaceutically acceptable excipients and/or auxiliaries.

6. The pharmaceutical composition of claim 5, wherein the additional API is selected from etoposide, fludarabine, mitoxantrone, methotrexate, prednisone, rituximab, vincristine and/or 90Y-ibritumomab tiuxetan.

7. A method of producing a pharmaceutical composition comprising bendamustine or a derivative thereof as an API comprising the steps of:
- mixing and melting the API and a polymer in order to provide a melt,
- extruding the melt through a die thereby forming an extrudate; and
- cooling the extrudate until it solidifies.

8. The method according to claim 7, wherein the API is admixed with the polymer before, during or after melting said polymer.

9. The method according to claim 7, wherein at least one pharmaceutically acceptable excipient and/or auxiliary is dispersed or dissolved in the polymer.

10. The method of one or more of claims 7 to 9 wherein the melt is formed at between 50 °C - 200 °C.

11. A pharmaceutical composition obtainable by the method of one or more of claims 7 to 10.

12. The pharmaceutical composition of one or more of claims 1 - 6 or 11 for oral, topical, pulmonary, nasal, or parenteral use.

13. The pharmaceutical composition of claim 11 or 12 for use in the treatment of neoplastic or autoimmune diseases.
